# EUROPÄISCHE PATENTSCHRIFT

(11) **EP 0 996 394 B1**
(45) Veröffentlichungstag und Bekanntmachung des Hinweises auf die Patenterteilung: **19.09.2007**
(21) Anmeldenummer: 98943692.8
(22) Anmeldetag: 14.07.1998
(51) Int. Cl.: A61F 2/30

(54) **KOMPRESSIONS-ENDOPROTHESENSYSTEM**
COMPRESSION ENDOPROSTHESIS SYSTEM
SYSTEME D'ENDOPROTHESE A COMPRESSION

(30) Priorität: 14.07.1997 DE 19730073
(43) Veröffentlichungstag der Anmeldung: 03.05.2000
(73) Patentinhaber: Jansson, Volkmar, Dr., D-82205 Gilching (DE)
(72) Erfinder: Jansson, Volkmar, Dr., D-82205 Gilching (DE)
(74) Vertreter: Czybulka, Uwe
(86) Internationale Anmeldenummer: PCT/DE1998/001967
(87) Internationale Veröffentlichungsnummer: WO 1999/003429

(56) Entgegenhaltungen:
- EP-A- 0 093 378
- EP-A- 0 112 435
- EP-A- 0 158 534
- EP-A- 0 196 258
- EP-A- 0 551 794
- EP-A- 0 696 439
- EP-A- 0 780 106
- WO-A-91/03992
- WO-A-91/16016
- DE-A- 4 437 479
- DE-A- 19 624 118
- DE-C- 3 630 069
- FR-A- 1 278 359
- FR-A- 2 651 674
- FR-A- 2 707 479
- GB-A- 2 159 416
- US-A- 5 326 368

## Beschreibung

Die Erfindung betrifft ein Endoprothesensystem für zementfreie Implantation gemäß dem Oberbegriff des Anspruchs 1.

In der Hüftendoprothetik stellen insbesondere die Wechseloperationen bei ausgelockerten Implantaten eine besondere Problematik dar. Beim Vorliegen ausgedehnter Knochendefekte sowohl im Pfannenlager als auch im Schaftbereich werden aufwendige Rekonstruktionen erforderlich. Dazu können im Pfannenbereich z.B. Abstützschalen verwendet werden, die z.B. mit Schrauben fixiert und mit Knochenzement und Knöchenspänen stabilisiert werden. Im Hüftschaftbereich lassen sich bei ausgedehnten proximalen Defekten stabile Verhältnisse oft nur mit weit distal im Oberschenkelknochen verankernden Prothesensystemen erzielen.

Eine interessante Alternative bei ausgedehnten Pfannen- und proximalen Schaftdefekten stellt die Auspolsterung dieser Defekte mit Knochenchips (autolog, homologe oder Knochenersatzwerkstoffe) dar (Gie GA, Linder L, Ling RSM, Simon J-P, Sloof TJJH, Timperley AJ (1993) Impacted cancellous allografts and cement for revision total hip arthroplasty. J Bone Joint Surg. Vol.75-B, No.1:14-21).

Zur Impaktierung der Spongiosaplastik wurde ein spezielles Instrumentarium entwickelt, mit dem eine Verdichtung der Knochenchips in die Defekte hinein erzielt werden kann (Gie GA, Linder L, Ling RSM, Simon J-P, Sloof TJJH, Timperley AJ (1993) Contained morselized allograft in revision total hip arthroplasty. Orthop Clin North Am. Vol.24, No.4:717-725).

Sowohl im Schaft als auch im Pfannenbereich kann in ein so derartig neu geschaffenes Knochenlager eine Pfanne oder ein Schaft einzementiert werden. Diese Technik ist mit klinischen Studien inzwischen gut abgesichert (Sloof TJJH, Buma P, Schreurs BW, Schimmel JW, Huiskes R, Gardeniers J (1996) Acetabular and Femoral Reconstruction With Impacted Graft and Cement. Clin Orthop Rel Res. No.323:108-115).

Nachteilig bei dieser Technik ist, dass sowohl im Schaft- als auch im Pfannenbereich zementierte Implantate verwendet werden müssen.

Aus der EP-A2-0780 106, die zu der Bildung des Oberbegriffes des Anspruches 1 herangezogen wurde, ist eine Endopropthese bekannt, die auch zementfrei zu implantieren ist und einen Schaft aufweist, der im proximalen Bereich medial abgerundet und lateral mit einer nach proximal sich erweiternden und im wesentlichen in Längsrichtung des Schaftes bzw. proximal leicht nach medial geneigten Rippe versehen ist. Ventral und dorsal ist der Schaft jeweils mit einer Rippe, die sich nach proximal aufweiten, sodass Kompressionsflanken gebildet werden, die einen nach proximal offenen Winkel bilden. Es kann auch ein Rippensystem aus niedrigen, sich nach proximal aufweitenden Stufenflächen vorgesehen sein. Die Seitenränder der Stufenflächen verlaufen im Wesentlichen in Richtung medial.

Die in die Prothese beim Gebrauch eingeleiteten Kräfte erfolgen relativ einseitig in leicht lateral geneigter Richtung, sodass bei einer zementfreien Implantation auf Dauer eine Lockerung stattfinden wird. Aus diesem Grunde wird diese Prothese in der Regel in den Femur einzementiert.

Allgemein scheinen zementfreie Prothesen keine ausreichende Stabilität im ncu geschaffenen Knochenlager zu finden.

Es ist Aufgabe der Erfindung, mit Hilfe eines speziellen Prothesendesigns eine ausreichend feste Verankerung eines zementfreien Implantes in einem neu geschaffenen Lager aus Knochenchips zu erzielen.

Diese Aufgabe wird gemäß der Erfindung durch die Merkmale des Patentanspruches 1 gelöst.

Weitere Ausgestaltungen der Erfindung gehen aus den Unteransprüchen hervor.

Demgemäß weist das Endoprothesensystem an dem Prothesenschaft eine einzige ventrale und eine einzige dorsale Rippe auf, wobei der Querschnitt jeder Rippe an der Osteotomieebene sich gegenüber dem Querschnitt der Rippe am proximalen Drittel um wenigstens den Faktor 1,5 aufweitet; die Rippentiefe der einzelnen Rippen beträgt hierbei im proximalen Drittel mindestens 10% der Dicke des Prothesengrundkörpers.

Die Rippen bilden einen nach proximal offenen Winkel, wobei die lateralen Seitenränder nach lateral und die medialen Seitenränder nach medial geneigt sind. Diese tief gestuften Seitenränder wirken auf diese Weise als Kompressionsflanken.

Die auf das Hüftgelenk einwirkenden Gelenkkräfte werden auf diese Weise so in das neu geschaffene Lager aus Knochenchips eingeleitet, dass die Knochenchips durch die auf sie wirkende Kraft komprimiert werden und sich dabei gegeneinander und gegen die Knochenwandung des Hohlknochens anpressen.

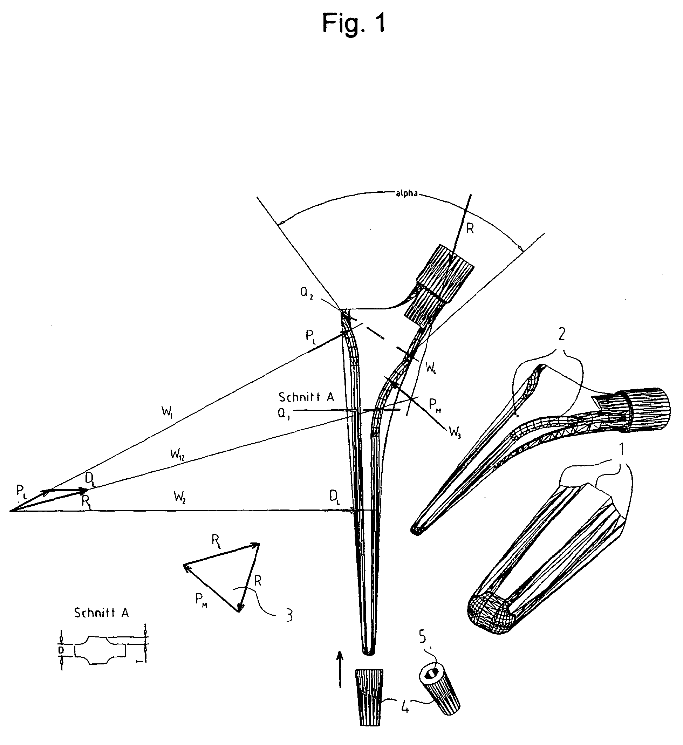

Im Fall des Hüftgelenkes führt die einwirkende Kraft (Pauwels F(1973) Atlas zur Biomechanik der gesunden und kranken Hüfte. Springer-Verlag Berlin, Heidelberg, New York) als "Hüftresultierende" (R, s. Fig. 1) im Wesntlichen zu einer axialen Belastung des Hüftschaftes. Ziel des Prothesendesigns ist es also, durch eine proximale Aufweitung des Schaftes eine Verdichtung der Knochenchips zu erzielen. Dieses ist mit der oben diskutierten zementfreien Prothese gemäß der EP-A2-0 780106 nicht möglich.

Fig. 1 zeigt am Beispiel eines Hüftendoprothesenschaftes, wie eine solche proximale Kompression erzielt werden kann. Dazu wird im proximalen Schaftbereich ein "Rippensystem" (1) eingebracht. Mindestens eine dieser Rippen weitet sich nach proximal so weit auf, dass sich im proximalen Schaftbereich "Kompressionsflanken" (2) bilden, so dass eine axiale Belastung der Prothese zu einer maximalen Kompression und Verdichtung der um die Prothese herum im Knochenlager angelagerten Knochenchips führt, so dass ein sicheres Verklemmen der Prothese in diesem Knochenlager gewährleistet ist. Die prinzipielle Krafteinleitung in das Femur unter Vernachlässigung der Reibung und eines geschätzen Angriffspunktes der Kraft D_{L} ist im Kraftgleichgewicht (3) der Fig. 1 dargestellt.

Um dieses sicher zu erreichen ist zu fordern, dass das Verhältnis der Rippentiefe T (der die Kompressionsflanken bildenden Rippen) zu der Prothesendicke D (Fig.1) (im proximalen Drittel) mindestens T/D = 0.1 beträgt, und dass das Verhältnis der Querschnitte der (die Kompressionsflanken bildenden) Rippen am proximalen Drittel Q₁ zu dem Querschnitt der Rippen an der Osteotomieebene Q₂ mindestens Q₂/Q₁ = 1.5 beträgt.

Dabei ist die Art der Aufweitung nicht unerheblich. Wenn, wie in Fig. 1 gezeigt, durch eine gebogene Rippenstruktur eine progressive Aufweitung erzielt werden kann, so wird der Effekt der Kompression extrem weit nach proximal verlagert. Die Krafteinleitung in den Schaftknochen erfolgt somit ebenfalls sehr proximal, was aus biomechanischer Sicht eine besonders günstige Krafteinleitung bedeutet. Begünstigt wird dieses noch durch den nach proximal offenen Winkel (alpha, s. Fig. 1) der Kompressionsflanken.

Eine wie in der Fig. 1 gestaltete Prothese ist daher auch zu Primärimplantation besonders gut geeignet, da - auch ohne eine sonst übliche Kragenkonstruktion - eine extrem proximale Krafteinleitung erzielt werden kann. Da sich diese Krafteinleitung - im Gegensatz zu einem Prothesenkragen - über eine längere Distanz erstreckt, ist auch das grundsätzliche Problem des Prothesenkragens (schlechter primärer Aufsitz, sekundäres Wegweichen des Knochens vom Kragen infolge der lokal zu großen Lasteinleitung) gelöst. Eine wie in Fig. 1 gestaltete Schaftprothese erfüllt somit die Funktion eines "kragenlosen Kragens".

Durch die Rippenkonstruktion wird ein weiteres wichtiges Kriterium der Verankerung erfüllt: Insbesondere Hüftendoprothesenschäfte werden zusätzlich zu der Hüftresultierenden (Pauwels, s.o.) auch durch Rotationsmomente belastet. Diese Rotationskräfte können durch eine Rippenkonstruktion gemäß der Erfindung besonders gut aufgenommen werden, wobei die gleichzeitige Kompression des proximalen Schaftlagers eine zusätzliche Verklemmung der Prothese und Sicherung gegen Rotation bedeutet.

Bei Revisionsoperationen ist das proximale Schaftlager infolge der gelockerten Endoprothese stark geschädigt. Aufgrund der z.T. erheblichen Knochendefekte reicht oft eine reine proximale Verankerung der Prothese für einen stabilen Prothesensitz nicht aus. Um in einem solchen Fall trotzdem eine letztendlich proximale Verankerung der Schaftprothese erzielen zu können, muss - wenigstens temporär bis zum Einheilen der Knochenchips - eine distale Lasteinleitung in den Knochenschaft sichergestellt werden.

Dies wird gemäß der Erfindung durch einen "Markraumstopper" erfüllt, der zweierlei Aufgaben erfüllt. Zum einen dient er als Verschluss des Röhrenknochens, so dass die proximal von dem Stopper eingebrachten Knochenchips verdichtet werden können, so wie dies in "Gie GA, Linder L, Ling RSM, Simon J-P, Sloof TJJH, Timperley AJ (1993) Contained morselized allograft in revision total hip arthroplasty. Orthop Clin North Am. Vol.24, No.4:717-725" (s.o.) beschrieben ist. Zum anderen ist das proximale Ende des Markraumstoppers (4) jedoch so geformt, dass eine Abstützung der distalen Prothesenspitze erfolgen kann, so wie dieses in Fig.1 dargestellt ist. Dieses kann als "Aufsitz" zur reinen Einleitung der axialen Kräfte erfolgen, oder aber (s. Fig. 1) über eine formschlüssige Verbindung (5) zwischen Markraumstopper und Prothesenspitze, so dass auch die Übertragung von Biege- und Torsionsmomenten erfolgen kann. In diesem Fall wirkt der Markraumstopper wie eine nach distal verlängerte Prothese.

Besonders vorteilhaft ist es dabei, wenn der Markraumstopper aus einem resorbierbaren Material (ggf. auch in offenporiger Struktur zur Minimierung des vom Körper abzubauenden Materials) hergestellt ist, so dass innerhalb einer bestimmten Zeit die Lasteinleitung in den distalen Knochenschaft ab- und in den proximalen Knochenschaft zunimmt. Als Material kommen dabei z.B. Polyglycolide oder Polylactide in Frage. Natürlich können auch andere Materialien wie Implantatmetall oder andere nicht abbaubare Kunststoffe (z.B. Polyethylen) verwendet werden.

Im Bereich der Pfannenverankerung finden sich die größten zu überbrückenden Defekte meist im cranialen Pfannenerkerbereich. Klinisch kommt es dadurch zu einem Höhertreten der Pfanne und zu einer entsprechenden Beinverkürzung. Bei einer Rekonstruktion muss daher insbesondere im lateralen Pfannenerkerbereich ein mechanisch stabiles Lager geschaffen werden, so dass die neu implantierte Pfanne distalisiert werden kann. Ein derartiger Pfannenaufbau unter Verwendung von autologem oder homologem Knochen bzw. Knochenersatzmaterialien ist jedoch nicht unproblematisch: Werden z.B. große stabile Fremdknochenblöcke verwendet (die für die mechanische Stabilität wünschenswert wären), so besteht die Gefahr, dass diese großen Knochenstücke nicht knöchern umgebaut werden und auf Dauer mechanisch versagen oder resorbiert werden. Werden jedoch kleine Spongiosa- oder Knochenersatzmaterialchips verwendet (die besser in körpereigenen Knochen umgebaut werden), so besteht die Gefahr, dass sich die aus diesen Stückchen geformte Masse nicht genügend fest in die Knochendefekte einpressen läßt und aus den Defekten wieder löst.

Dieses Problem wird gemäß der Erfindung dadurch gelöst, dass eine spezielle press-fit-Pfanne mit einem asymmetrischen Rippensystem (6) versehen wird. Diese Rippen ragen in den - meist lateral gelegenen - Pfannendefekt hinein, erreichen bei kleineren Knochendefekten sogar den Knochen des lateralen Knochendefektes im Wirtslager und bilden "Kammern" (8), in die kleine Spongiosa- oder Knochenersatzmaterialchips eingebolzt werden können.

Besonders vorteilhaft ist es auch, wenn dieses Rippensystem im lateralen Pfannenerkerbereich aus resorbierbaren Materialien im Sinne von Hilfsrippen (9) hergestellt wird, so dass nach Abbau dieses Materials eine relativ kleine Metallpfanne im knöchernen Lager verbleibt. Dabei ist es sehr günstig, wenn verschieden große Hilfsrippen intraoperativ mit der Pfanne und ggf. auch mit dem Einschlagintrumentarium (10) über Nut- und Steckverbindungen (11,12) verbunden werden können, so dass eine individuelle Anpassung der Pfanne an die knöchernen Gegebenheiten erfolgen kann.

Dabei kann es auch sehr vorteilhaft sein, wenn dieses Kammersystem (besonders vorteilhaft aus resorbierbarem Material hergestellt) im Sinne eines "Kraftträger und Platzhalters" (13) von der Pfanne getrennt, aber der Pfannengeometrie angepasst (17) ist und mit der Pfanne verbunden werden kann: Dadurch können durch Wahl verschieden großer Kraftträger und Platzhalter unterschiedlich große Defekte im lateralen Pfannenerkerbereich aber auch in der weiteren Zirkumferenz gedeckt werden. Durch Wahl verschieden großer Kraftträger und Platzhalter kann so eine individuelle Anpassung des Pfannensystems an die vorliegenden Knochendefekte erzielt werden.

Natürlich muss ein solcher Kraftträger und Platzhalter (13) z.B. durch Querstege (15) in sich stabilisiert werden, wobei auf eine größtmögliche Offenporigkeit geachtet werden muss, damit die in die Zwischenräume (16) eingebolzten kleinen Spongiosa- oder Knochenersatzmaterialchips auch Anschluss an den Wirtsknochen finden und ein- und umgebaut werden können.

Die folgenden Abbildungen erläutern einige bevorzugte Ausführungen der Schaft- und Pfannenprothese.
- Fig. 1: zeigt einen Hüftendoprothesenschaft mit einem sich nach proximal extrem aufweitenden Rippensystem mit 4 Rippen (1). Die ventrale und dorsale Rippe weiten sich im proximalen Drittel auf und bilden die Kompressionsflanken (2), die einen nach proximal offenen Winkel (alpha) bilden, wobei die laterale Flanke nach lateral und die mediale Flanke nach medial geneigt ist und über die die Verdichtung der Spongiosa des knöchernen Lagers (beim Prothesenwechsel: die angelagerten Spongiosachips) erfolgt. Das idealisierte Kraftgleichgewicht (3) erläutert die prinzipielle Krafteinleitung in das Knochenlager: Der Hüftresultierenden R halten die Kräfte P_{L}, P_{M} und D_{L} das Gleichgewicht. Über die Kräfte P_{L} und P_{M} kommt es zu der gewünschten Kompression des Knochenlagers. Die distale Abstützung der Prothese bei ausgedehnten proximalen Knochendefekten kann über den Markraumstopper (4) erfolgen. Über einen Formschluss (5) können so sogar Biege- und Torsionsmomente übertragen werden.

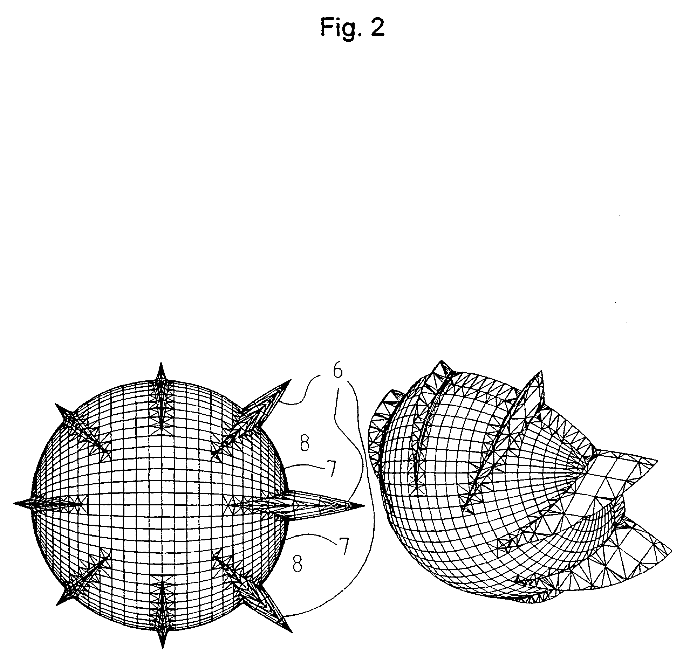

- Fig. 2: zeigt eine Hüftendoprothesenpfanne mit einem asymmetrischen fest mit der Pfanne verbundenen Rippensystem, das in den - meist im latero/cranialen Pfannenerkerbereich gelegenen - Knochendefekt hineinragt. In die von den Rippen (6), der Pfannenoberfläche (7) und der Knochenwand des Knochendefektes gebildeten "Kammern" (8) können kleine Spongiosa- oder Knochenersatzmaterialchips eingebolzt werden.

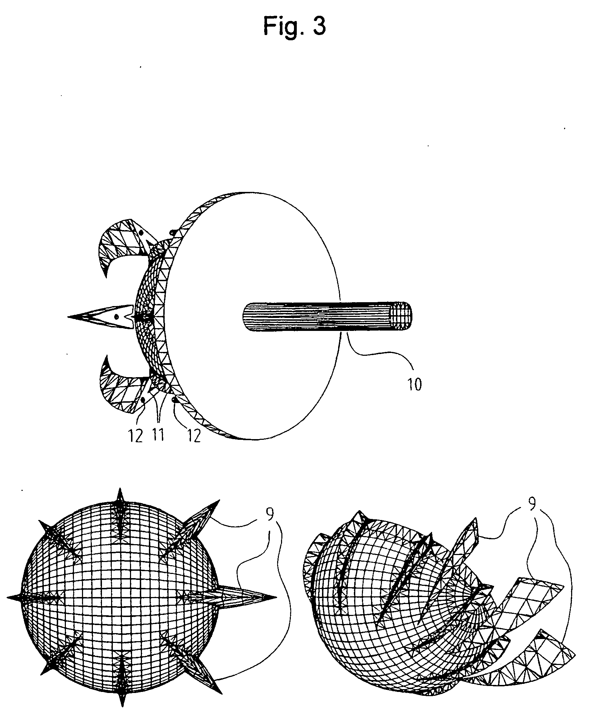

- Fig. 3: zeigt eine Hüftendoprothesenpfanne mit einem asymmetrischen Rippensystem, bei dem die die Asymmetrie verursachenden Hilfsrippen (9) mit der Pfanne - z.B. durch ein hier nicht dargestelltes Schwalbenschwanzprofil - intraoperativ mit der Pfanne verbunden werden, so dass durch Wahl verschieden großer Rippen eine individuelle Anpassung an die Knochendefekte des Wirtsknochens erfolgen kann. Eine besonders gute Verbindung erfolgt auch dann, wenn - wie hier dargestellt - die Hilfsrippen an ihrer Basis eine der Rippenform der Pfanne angepasste Nut (11) aufweisen und über eine Steckverbindung (12) an dem Einschlaginstrumentarium (10) lösbar verbunden werden können, so dass während des Einschlagens der Pfanne in das Knochenlager eine feste Verbindung der Hilfsrippen mit der Pfanne gewährleistet ist.

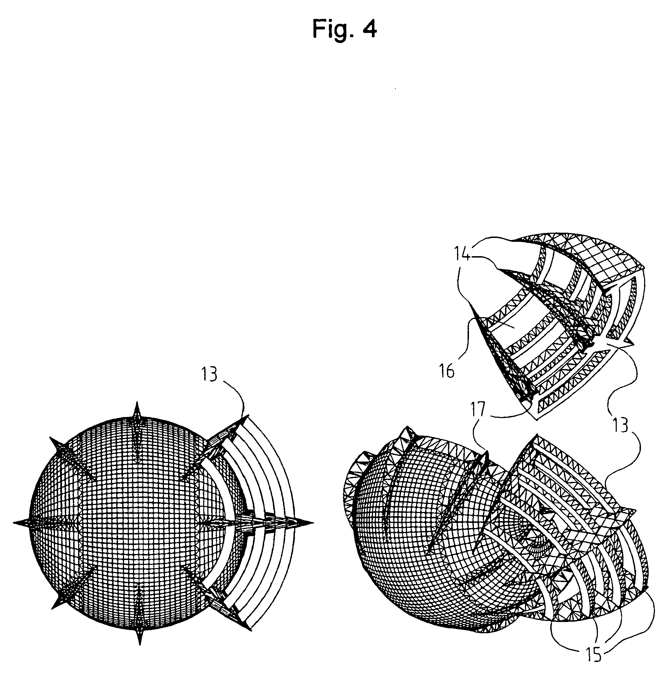

- Fig. 4: zeigt eine Hüftendoprothesenpfanne mit einem angelagerten offenporigen Kraftträger und Platzhalter (13), der in die Defekte zwischen Wirtsknochen und Pfanne eingebracht wird und so eine Verbindung zwischen Wirtsknochen und Pfanne herstellt. Natürlich können auch mehrere derartige Kraftträger und Platzhalter - auch unterschiedlicher Größe - verwendet werden, um eine individuelle Anpassung der Defekte an die knöchernen Gegebenheiten zu erreichen.
Zur Verstärkung der mechanischen Eigenschaften verfügt der Kraftträger und Platzhalter außer den Rippen (14) auch über einige Querstege (15). In die von Rippen und Querstegen gebildeten Zwischenräume (16) können kleine Spongiosa- oder Knochenersatzmaterialchips eingebolzt werden. Durch eine geometrische Anpassung des Kraftträger und Platzhalters (17) an die Pfanne wird eine Verbindung zwischen Kraftträger und Platzhalter und Pfanne erzielt.

## Patentansprüche

1. Endoprothesensystem für zementfreie Implantation mit einem Prothesenschaft, der eine einzige ventrale und eine einzige dorsale Rippe (1) aufweist, welche Rippen sich jeweils nach proximal aufweiten, so dass Kompressionsflanken (2) gebildet werden, die einen nach proximal offenen Winkel (alpha) bilden, **dadurch gekennzeichnet, dass** jeweils der Querschnitt (Q2) der Rippe an der Osteotomieebene sich gegenüber dem Querschnitt (Q1) der Rippe am proximalen Drittel um wenigstens den Faktor 1,5 aufweitet, dass die Rippentiefe (T) der einzelnen Rippen im proximalen Drittel mindestens 10 % der Dicke des Prothesengrundkörpers beträgt, und dass die lateralen Kompressionsflanken der Rippen nach lateral und die medialen Kompressionsflanken der Rippen nach medial geneigt sind.

2. Endoprothesensystem nach Anspruch 1, **dadurch gekennzeichnet, dass** das Rippensystem vier Rippen (1) aufweist.

3. Endoprothesensystem nach einem der vorhergehenden Ansprüche, **dadurch gekennzeichnet, dass** der Prothesenschaft über einen distalen Markraumstopper (4) aus resorbierbarem Werkstoff verfügt, mit dem der Schaft bei oder vor der Implantation formschlüssig oder im Sinne eines Aufsitzes verbunden werden kann, so dass ein Teil der auf das Gelenk einwirkenden Last temporär über den Markraumstopper in den distalen Knochen eingeleitet wird.

4. Endoprothesensystem nach Anspruch 3, **dadurch gekennzeichnet, dass** der Markraumstopper aus dem Vollen oder aber porös gefertigt ist.

5. Endoprothesensystem nach einem der vorhergehenden Ansprüche, **dadurch gekennzeichnet, dass** dem Prothesenschaft eine Pfanne zugeordnet ist, und dass die Pfanne über ein asymmetrisches Rippensystem (6, 9) mit unterschiedlich großen Rippen verfügt, wobei mindestens eine Rippe in den zu deckenden Pfannendefekt des Wirtsknochens hereinragt.

6. Endoprothesensystem nach Anspruch 5, **dadurch gekennzeichnet, dass** in die Knochendefekte hineinragende Hilfsrippen (9) zunächst von der Pfanne getrennt sind und mit der Pfanne fest oder lösbar verbindbar sind, sodass intraoperativ eine individuelle Anpassung der Pfanne an die knöchernen Defekte erzielt werden kann.

7. Endoprothesensystem nach Anspruch 6, **dadurch gekennzeichnet, dass** die Hilfsrippen über Nut- und Steckverbindungen (11,12) mit der Pfanne und lösbar auch mit einem Einschlaginstrumentarium verbindbar sind.

8. Endoprothesensystem nach Anspruch 6 oder 7, **dadurch gekennzeichnet, dass** die mit der Pfanne zu verbindenden Rippen aus einem resorbierbaren Material, aus nicht resorbierbarem Kunststoff oder aus Implantatmetall als Vollmaterial oder offenporig hergestellt sind.

9. Endoprothesensystem nach Anspruch 5, für zementfreie Implantation, bestehend aus Schaft und Pfanne, **dadurch gekennzeichnet, dass** die knöchernen Defekte zwischen Pfanne und Wirtsknochen mit zumindest einem Kraftträger und Platzhalter (13, 17) gedeckt werden, die eine offenporige Verbindung zwischen Wirtsknochen und Pfanne herstellen.

10. Endoprothesensystem nach Anspruch 9, **dadurch gekennzeichnet, dass** der Kraftträger und Platzhalter aus einem resorbierbaren Material, einem nicht resorbierbaren Kunststoff oder aus Implantatmetall hergestellt sind.

## Claims

1. An endoprothesis system for a cement-free implantation comprising a prothesis shaft which comprises a single ventral and a single dorsal rib (1), said ribs expanding in each case in the proximal direction so that compression flanks (2) are formed which form an angle (alpha) that is open in the proximal direction, **characterized in that** the cross-section (Q2) of the rib at the osteotomy plane is expanded by at least the factor of 1.5 relative to the cross-section (Q1) of the rib at the proximal third, that the depth of the ribs (T) of the individual ribs is at least 10% of the thickness of the basic body of the prothesis in the proximal third and that the lateral compression flanks of the ribs are inclined in the lateral direction and the medial compression flanks of the ribs are inclined in the medial direction.

2. The endoprothesis system according to claim 1, **characterized in that** the rib system comprises four ribs (1).

3. The endoprothesis system according to any of the preceding claims, **characterized in that** the prothesis shaft comprises a distal medullary space stopper (4) made of a resorbable material, with which the shaft can be connected during or prior to the implantation in a positive locking fashion or in the sense of an abutment so that part of the load acting on the joint is temporarily introduced into the distal bone via the medullary space stopper.

4. The endoprothesis system according to claim 3, **characterized in that** the medullary space stopper is manufactured from the solid material or, however, in a porous fashion.

5. The endoprothesis system according to any of the preceding claims, **characterized in that** an acetabulum is allocated to the prothesis shaft and that the acetabulum comprises an asymmetrical rib system (6, 9) with differently large ribs, at least one rib projecting into the acetabulum defect of the host bone, which is to be covered.

6. The endoprothesis system according to claim 5, **characterized in that** auxiliary ribs (9) projecting into the bone defects are at first separated from the acetabulum and can be firmly or detachably connected with the acetabulum so that an individual adaptation of the acetabulum to the osseous defects can be achieved during the operation.

7. The endoprothesis system according to claim 6, **characterized in that** the auxiliary ribs can be connected with the acetabulum through groove-and-plug connections and, detachably, also with drive-in means.

8. The endoprothesis system according to claim 6 or 7, **characterized in that** the ribs to be connected with the acetabulum are made from a resorbable material, from a non-resorbable plastic material or from implant metal as a solid or open-pore material.

9. The endoprothesis according to claim 5 for a cement-free implantation that consists of a shaft and an acetabulum, **characterized in that** osseous defects between the acetabulum and the host bone are covered with at least one force absorber and a spacer which establish an open-pore connection between the host bone and the acetabulum.

10. The endoprothesis system according to claim 9, **characterized in that** the force absorber and spacer are made from a resorbable material, a non-resorbable material or an implant metal.

## Revendications

1. Système d'endoprothèse pour implantation sans ciment, comportant un fût de prothèse qui présente une seule nervure (1) ventrale et une seule nervure (1) dorsale, nervures qui s'évasent chacune en direction proximale, de manière à réaliser des flancs de compression (2) qui forment un angle (alpha) ouvert en direction proximale, **caractérisé en ce que** la section transversale (Q2) de chaque nervure sur le plan d'ostéotomie s'évase d'au moins le facteur 1,5 par rapport à la section transversale (Q1) de la nervure sur le tiers proximal, **en ce que** la profondeur de nervure (T) des nervures individuelles dans le tiers proximal est d'au moins 10 % de l'épaisseur du corps de base de prothèse, et **en ce que** les flancs de compression latéraux des nervures sont inclinés en direction latérale et les flancs de compression médians des nervures sont inclinés en direction médiane.

2. Système d'endoprothèse selon la revendication 1, **caractérisé en ce que** le système de nervures présente quatre nervures (1).

3. Système d'endoprothèse selon l'une des revendications précédentes, **caractérisé en ce que** le fût de prothèse dispose d'un bouchon intramédullaire (4) distal en substance résorbable, avec lequel le fût peut être relié par coopération de formes ou dans le sens d'une venue en butée lors ou avant l'implantation, de telle sorte qu'une partie de la charge agissant sur l'articulation est temporairement introduite dans l'os distal via le bouchon intramédullaire.

4. Système d'endoprothèse selon la revendication 3, **caractérisé en ce que** le bouchon intramédullaire est réalisé dans la masse ou poreux.

5. Système d'endoprothèse selon l'une des revendications précédentes, **caractérisé en ce qu'**au fût de prothèse est associée une cupule, et **en ce que** la cupule dispose d'un système de nervures (6, 9) asymétriques avec des nervures de différentes tailles, au moins une nervure faisant saillie dans le défaut de cupule de l'os receveur.

6. Système d'endoprothèse selon la revendication 5, **caractérisé en ce que** des nervures auxiliaires (9) faisant saillie dans les défauts osseux sont tout d'abord séparées de la cupule et peuvent être reliées à la cupule de manière solidaire ou détachable, de telle sorte que l'on peut obtenir de manière intraopératoire une adaptation individuelle de la cupule aux défauts osseux.

7. Système d'endoprothèse selon la revendication 6, **caractérisé en ce que** les nervures auxiliaires peuvent être reliées via des liaisons à rainures et par enfichage (11, 12) à la cupule et de manière détachable également avec un instrument de frappe.

8. Système d'endoprothèse selon la revendication 6 ou 7, **caractérisé en ce que** les nervures à relier avec la cupule sont réalisées en un matériau résorbable, en matière plastique non résorbable ou en métal d'implant, en tant que matériau plein ou à pores ouverts.

9. Système d'endoprothèse selon la revendication 5, pour l'implantation sans ciment, constitué par un fût et une cupule, **caractérisé en ce que** les défauts osseux entre la cupule et l'os receveur sont couverts par au moins un support de force et un mainteneur d'espace (13, 17) qui établissent une liaison à pores ouverts entre l'os receveur et la cupule.

10. Système d'endoprothèse selon la revendication 9, **caractérisé en ce que** le support de force et le mainteneur d'espace sont réalisés en un matériau résorbable, en une matière plastique non résorbable ou en un métal d'implant.
